# EUROPÄISCHE PATENTSCHRIFT

(11) **EP 1 991 160 B1**
(45) Veröffentlichungstag und Bekanntmachung des Hinweises auf die Patenterteilung: **08.05.2019**
(21) Anmeldenummer: 07723029.0
(22) Anmeldetag: 03.03.2007
(51) Int. Cl.: A61F 2/00

(54) **FLÄCHIGES IMPLANTAT, INSBESONDERE HERNIENNETZ**
FLAT IMPLANT, PARTICULARLY A HERNIA MESH
IMPLANT EN NAPPE, NOTAMMENT FILET POUR HERNIE

(30) Priorität: 09.03.2006 DE 102006011903
(43) Veröffentlichungstag der Anmeldung: 19.11.2008
(73) Patentinhaber: Aesculap AG, 78532 Tuttlingen (DE)
(72) Erfinder: ABELE, Wolfgang, 78532 Tuttlingen (DE); KUPFERSCHMID, Franz-Josef, 78576 Emmingen-Liptingen (DE); ODERMATT, Erich, 8200 Schaffhausen (CH); MÜLLER, Erhard, 70565 Stuttgart (DE); SCHMEES, Hans-Gerd, 72827 Wannweil (DE)
(74) Vertreter: Patentanwälte Ruff, Wilhelm, Beier, Dauster & Partner mbB
(86) Internationale Anmeldenummer: PCT/EP2007/001838
(87) Internationale Veröffentlichungsnummer: WO 2007/101630

(56) Entgegenhaltungen:
- EP-A- 1 600 118
- WO-A-01/15625
- WO-A-02/35984

## Beschreibung

Die Erfindung betrifft ein flächiges Implantat, insbesondere ein Herniennetz, in Form eines Öffnungen aufweisenden textilen Netzes aus nicht resorbierbaren monofilen Fäden.

Herniennetze sind in verschiedenen Ausstattungen bekannt. Gewöhnlich handelt es sich um flächige grobmaschige Gewirke aus monofilen Polypropylenfäden.

Herniennetze werden normalerweise durch kleinere Bauchöffnungen eingeführt und sollen sich im Bauchinnern möglichst in einfacher Weise entfalten und an die Bauchinnenwand anlegen lassen oder in Zwischenschichten einbetten lassen. Auf der einen Seite ist es erwünscht, möglichst wenig Fremdmaterial zu implantieren, weil es sich gezeigt hat, dass sich dadurch unerwünschte Abwehrreaktionen und Vernarbungen eher vermeiden lassen als bei relativ steifen Herniennetzen mit großem Flächengewicht. Auf der anderen Seite können bei Herniennetzen mit geringem Flächengewicht, d.h. solchen aus dünnen monofilen Fäden, Probleme beim Entfalten auftreten, weil solche Herniennetze ein geringes Rückstellvermögen aufweisen und sich nach dem Einbringen in die Bauchhöhle nicht leicht entfalten.

Um hier Problemlösungen zu finden, wurden kombinierte Herniennetze entwickelt, die aus einer Grundstruktur, die in der Regel aus nicht resorbierbarem Material besteht, und aus einer überlagerten Verstärkungsstruktur, die in der Regel aus resorbierbarem Material besteht, zusammengesetzt sind. Solche unabhängig voneinander bestehenden Wirkstrukturen werden in der Regel auf ein- oder zweifonturigen Maschinen hergestellt und sind beispielsweise aus der EP 0 797 962 B1, der EP 1 099 422 B1 sowie der DE 199 42 611 C1 bekannt. Herniennetze, die aus einem Verbund aus resorbierbaren und nicht resorbierbaren Fäden bestehen, haben den Vorteil, dass sie die für die Implantation gewünschte Steifigkeit besitzen. Nach Implantation werden die resorbierbaren Fäden abgebaut, so dass nur noch das relativ leichte Gewirk aus nicht resorbierbaren Fäden übrigbleibt. Da die Verbundgewirke aus zwei selbständigen Gewirken bestehen, die beim Wirken miteinander verbunden werden, bleibt nach der Resorption der resorbierbaren Fäden ein intaktes Gewirk aus nicht resorbierbaren Fäden übrig. Allerdings haben derartige Verbundgewirke den Nachteil, dass sie in der Herstellung kompliziert sind.

Aus der WO 02/35984 A2 ist ein flächiges Implantat bekannt, welches Fäden verschiedener Fadenstärke aufweist.

Gegenstand der EP 1 600 118 A1 ist ein Implantat zur Suspension der Harnblase bei Senkungsbeschwerden und/oder bei Harninkontinenz der Frau. Das Implantat weist eine etwa rechteckige, ovale oder langgestreckt trapezförmige Basis auf, von deren Längsseiten in einem Abstand voneinander bandförmige Fortsätze abstehen, wobei die bandförmigen Fortsätze die gleiche Bindungsart aufweisen wie die Basis und mit dieser aus einem Stück bestehen.

Ein netzförmiges Implantat mit textilen Verstärkungselementen wird in der WO 01/15625 A1 beschrieben.

Das erfindungsgemäße Netz ist dadurch gekennzeichnet, dass es Fäden verschiedener Fadenstärke aufweist, die parallel zueinander verlaufen. Gemäß der Erfindung ist es somit vorgesehen, dass dickere und dünnere monofile Fäden, jeweils aus nicht resorbierbarem Material, im Netz verarbeitet sind. Die dickeren Fäden bringen die notwendige Steifigkeit, die eine gute Entfaltung ermöglicht, die inneren Fäden sorgen für die gewünschte Verringerung an der Menge des zu implantierenden Fremdstoffes. Mit parallelem Verlauf ist die Grobstruktur des textilen Materials zu verstehen. In den von der jeweiligen textilen Bindung abhängigen Feinstruktur können Abweichungen zum parallelen Verlauf vorliegen.

Es hat sich überraschenderweise gezeigt, dass es nicht erforderlich ist, die stärkeren und damit steiferen Fäden vollflächig im Netz vorzusehen. Vielmehr reicht es aus, wenn sogenannte Versteifungsrippen im Netz vorgesehen sind, die die Entfaltbarkeit begünstigen.

Bei den erfindungsgemäßen Netzen handelt es sich in der Regel um solche, die thermofixiert sind. Dadurch wird unter anderem ein unerwünschtes Ausfranzen verhindert. Die Thermofixierung erfolgt am gespannten Netz unter Erwärmung auf Temperaturen unterhalb des Erweichungsbereichs des polymeren Materials. Versteifungen, die zusätzlich zu den dickeren Fäden oder anstelle dieser vorgesehen sein können, können auch dadurch ausgebildet sein, dass die Thermofixierung streifenförmig verstärkt ist und die monofilen Fäden an Kreuzungsstellen insbesondere miteinander versintert sind. Solche Thermofixierungsstreifen können in beliebiger Richtung verlaufen, z.B. längs, quer und/oder diagonal. Sie können sich auch überkreuzen. Auch eine ringförmige Ausbildung ist möglich. Die Thermofixierungsstreifen können durch Auflegen von erwärmten Heißsiegelbalken erzeugt werden.

Mit besonderem Vorteil verlaufen die Fäden verschiedener Stärke im wesentlichen nur in einer Längsrichtung des textilen Netzes. Dadurch wird eine anisotrope Verstärkung erhalten, die für die Praxis völlig ausreicht.

Die Erfindung ist besonders geeignet für gewirkte Netze, insbesondere für Netze mit einer Gewirkstruktur in rechts-links Bindung. Dies bedeutet, dass die Fäden verschiedener Stärke in einer Gewirkstruktur innerhalb einer Bindung liegen, d.h. das erfindungsgemäße Netz kann in einfacher Weise wie ein an sich bekanntes rechts-links gewirktes Netz ausgebildet werden, wobei anstelle von Fäden gleicher Stärke Fäden verschiedener Stärke vorgesehen sind. Die Fäden verschiedener Stärke liegen somit vorzugsweise in der gleichen textilen Bindung vor und wechseln sich in der Bindung des Netzes gegenseitig ab. Normalerweise reicht es aus, wenn im textilen Netz nur zwei verschiedene Fadenstärken vorgesehen sind, was bevorzugt ist.

Bevorzugt verlaufen mindestens zwei Fäden einer Fadenstärke nebeneinander. Dies bedeutet, dass Fäden gleicher Stärke Längsstreifen im Gewirk bilden, die bevorzugt eine unterschiedliche Breite besitzen. Die Anzahl an Fäden mit geringerer Stärke ist vorzugsweise größer als die der Fäden mit größerer Stärke. Auf diese Weise kann die gewünschte Gewichtsverminderung erreicht werden. Das Zahlenverhältnis von Fäden geringerer Stärke zu Fäden größerer Stärke liegt bei 10:1 bis 2:1. So können zwischen zwei Verstärkungsfäden, d.h. Fäden größerer Fadenstärke, 10 dünnere Fäden verlaufen. Es ist aber beispielsweise auch möglich, 2 oder mehr Verstärkungsfäden parallel nebeneinander laufen zu lassen und dazwischen beispielsweise 5 bis 20 Fäden kleinerer Stärke anzuordnen.

Die Erfindung ermöglicht es durch eine unregelmäßige Anordnung der verschiedenen Fäden, beispielsweise durch variierende Abstände oder unterschiedliche Anzahl parallel nebeneinander verlaufender Fäden gleicher Stärke, die Steifigkeit des Herniennetzes bzw. seine Entfaltungstendenz in der Fläche variabel zu gestalten. Dies ist besonders dann vorteilhaft, wenn Herniennetze vorbestimmter Größe und vorbestimmter Eigenschaften vorgefertigt werden. Im Normalfall, d.h. in den Fällen, in denen der Chirurg eine gewünschte Größe des Herniennetzes aus einem großen vorgefertigten Netz ausschneidet, sind Netze mit über die Fläche gleichmäßigen Eigenschaften erwünscht. Insbesondere in solchen Fällen ist es daher bevorzugt, dass sich die Fäden mit größerer Stärke und die Fäden mit kleinerer Stärke in wiederholendem Rapport wechseln. Bevorzugt bestehen weiterhin die Fäden verschiedener Fadenstärke aus dem gleichen Material, auch wenn dies nicht zwingend ist. Als Materialien für die Fäden kommen insbesondere Polypropylen, Polyurethan, Polyethylenterephtalat und Polyvinylidendifluorid in Frage.

Die Fäden größerer Fadenstärke sind bei bevorzugten Ausführungsformen um 15 bis 60 %, insbesondere 20 bis 40 % dicker als die Fäden geringerer Fadenstärke. So haben sich stärkere Fäden mit einem Fadendurchmesser von 120 bis 200 µm, insbesondere 135 bis 165 µm, bewährt. Geeignete Fäden dünnerer Stärke haben vorzugsweise eine Fadenstärke von 80 bis 150 µm, insbesondere 110 bis 140 µm.

Die Netzöffnungen können in verschiedener Größe und Form ausgebildet sein. Die lichte Öffnung der Netzöffnungen liegt normalerweise zwischen 0,5 und 5 mm. Je nach Bindungsart, insbesondere Wirkart können die Öffnungen eine viereckige, insbesondere rautenförmige Form, eine fünfeckige, insbesondere diamantförmige Form, oder eine sechseckige, insbesondere wabenartige Form, aufweisen. Es sind auch Netze möglich, die abwechselnd verschiedene Öffnungsformen besitzen.

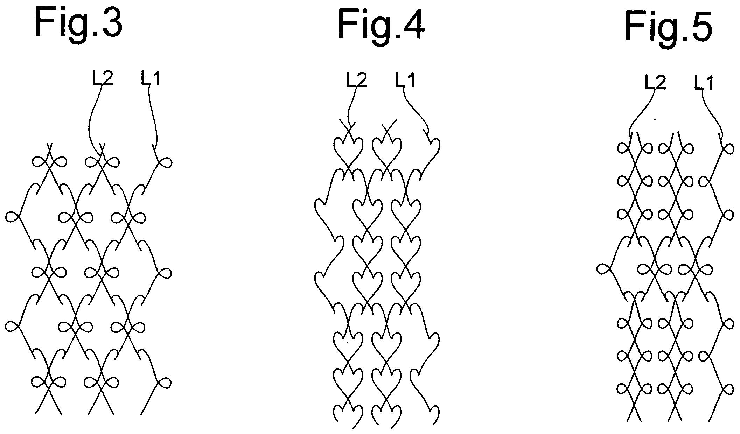

So kann ein Netz in Form eines Gewirkes mit einer Filetbindung und dem Legungsbild Figur 3 vorliegen, bei der die Öffnungen rautenförmig ausgebildet sind. Das Netz kann in Form eines Gewirkes mit einer Filetbindung und dem Legungsbild Figur 4 vorliegen, bei der die Öffnungen wabenförmig ausgebildet sind. Weiterhin kann das Netz mit einer Filetbindung und dem Legungsbild Figur 5 vorliegen, bei der sich in Längsrichtung verlaufende wabenförmige Öffnungsreihen und rautenförmige Öffnungsreihen gegenseitig abwechseln, wobei die Waben und Rauten in Längsrichtung gegeneinander versetzt sind. L1 und L2 in den Figuren 3 bis 5 bedeuten jeweils Legeschiene 1 und Legeschiene 2.

Die Breite der Verstärkungsstreifen kann je nach Anzahl der nebeneinander liegenden Verstärkungsfäden 2 bis 8 mm betragen, wobei hier ein unterer Bereich von 2 bis 4 mm bevorzugt ist, da sich gezeigt hat, dass Verstärkungsfäden als Einzelfäden bereits schon eine ausreichende Versteifung im Wirkverbund bringen. Die Breite der Streifen aus Fäden geringerer Fadenstärke liegt vorzugsweise bei 5 bis 25 mm, wobei hier ein oberer Bereich von 20 bis 25 mm bevorzugt ist.

Das Flächengewicht des erfindungsgemäßen Netzes kann zwischen 25 und 85 g/m² liegen. Das Flächengewicht des erfindungsgemäßen Netzes liegt mit Vorteil zwischen 30 und 80 g/m², wobei ein Flächengewicht von 35 bis 70 g/m² bevorzugt ist. Ganz bevorzugt sind Flächengewichte von 40 bis 65 g/m².

Die erfindungsgemäßen Netze können, um ihr Einwachsverhalten einerseits zu begünstigen und unerwünschte Adhäsionen auf der anderen Seite zu vermeiden, in geeigneter Weise vorbehandelt sein. So können die Fäden, insbesondere wenn sie aus hydrophobem Material bestehen, eine Oberflächenbeschichtung aufweisen oder hydrophil vorbehandelt sein, beispielsweise durch eine Plasmabehandlung. Um Adhäsionen zu vermeiden kann das Netz einseitig mit einer vollflächigen membranartigen Folie ausgerüstet, insbesondere beschichtet sein, die aus einem antiadhäsiven Material besteht. Weiterhin können die Netze sogenannte Guidelines aufweisen, insbesondere in Form von farbigen Längsstreifen. Hierzu bietet es sich an, die Verstärkungsfäden in anderer Farbe als das Grundmaterial einzusetzen. Aber auch Fäden dünnerer Stärke können eingefärbt sein.

Das Implantat kann auf der Oberfläche der Monofilamente mit wundheilungsfördernden und/oder antibiotisch wirkenden Stoffen versehen sein.

Weitere Merkmale der Erfindung ergeben sich aus der nachfolgenden Beschreibung von bevorzugten Ausführungsformen in Verbindung mit der Zeichnung und den Unteransprüchen. Hierbei können die einzelnen Merkmale jeweils für sich alleine oder zu mehreren in Kombination miteinander verwirklicht sein.

In den Zeichnungen zeigen:
- Figur 1:: eine Ausführungsform,
- Figur 2:: eine andere Ausführungsform,
- Figur 3:: die Bindung der Ausführungsform von Figur 1,
- Figur 4:: die Bindung einer Ausführungsform mit Wabenstruktur und
- Figur 5:: die Bindung der Ausführungsform von Figur 2.

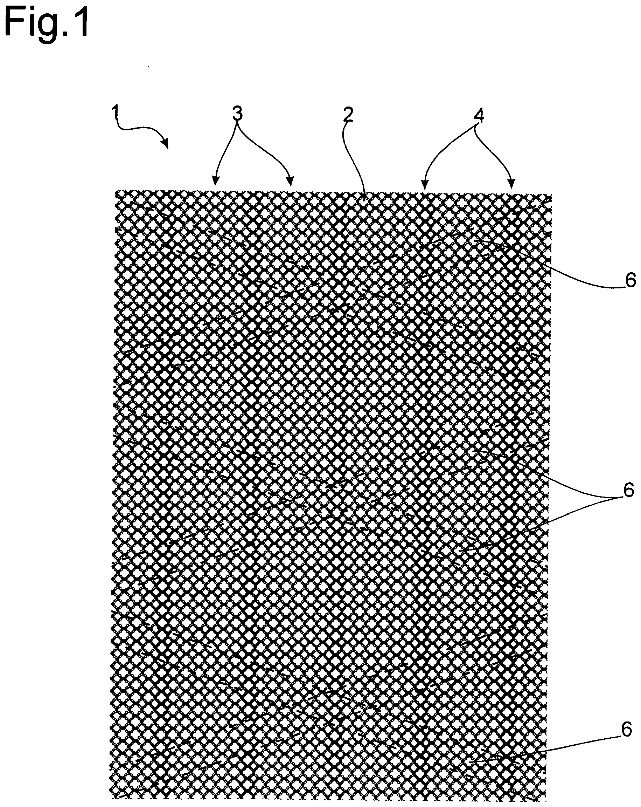

Bei der in der Zeichnung in Figur 1 dargestellten Ausführungsform der Erfindung ist ein thermofixiertes Herniennetz (1) als rechts-links Gewirk in einer Filetbindung und dem Legungsbild Figur 3 ausgebildet. Das Gewirk hat eine netzartige Struktur, in der rautenförmige Öffnungen (2) in Längsreihen angeordnet sind, wobei benachbarte Reihen jeweils auf Lücke versetzt sind. Die rautenförmigen Öffnungen besitzen eine lichte Maschenweite von ca. 3 mm. Das Gewirk ist streifenförmig ausgebildet, wobei sich breite Längsstreifen (3) mit schmalen Längsstreifen (4) regelmäßig abwechseln. Die breiten Längsstreifen bestehen aus monofilen Polypropylenfäden, die eine Fadenstärke von ca. 120 µm aufweisen.

Demgegenüber bestehen die schmalen Längsstreifen aus monofilen Polypropylenfäden mit einer größeren Fadenstärke von ca. 150 µm. Die schmalen Längsstreifen besitzen eine Breite von ca. 3 mm und die breiten Längsstreifen eine Breite von ca. 15 mm, was einem Fadenverhältnis von Fäden größerer Dicke zu Fäden kleinerer Dicke von 1:5 entspricht. Das Herniennetz mit den rautenförmigen Öffnungen besitzt in Längs- und Querrichtung eine weitgehend isometrische Dehnung und Bruchdehnung.

Die Fäden größerer Dicke, d.h. diejenigen, die die schmalen Streifen bilden, sind bei der dargestellten Ausführungsform dunkel gefärbt, wogegen die Fäden der schmalen Streifen farblos sind. Dadurch werden parallele Guidelines gebildet, die die Ausrichtung der Herniennetze bei der Implantation erleichtern. Figur 1 zeigt zusätzliche thermische Verstärkungen 6, die diagonal verlaufen und durch gestrichelte Linien angedeutet sind.

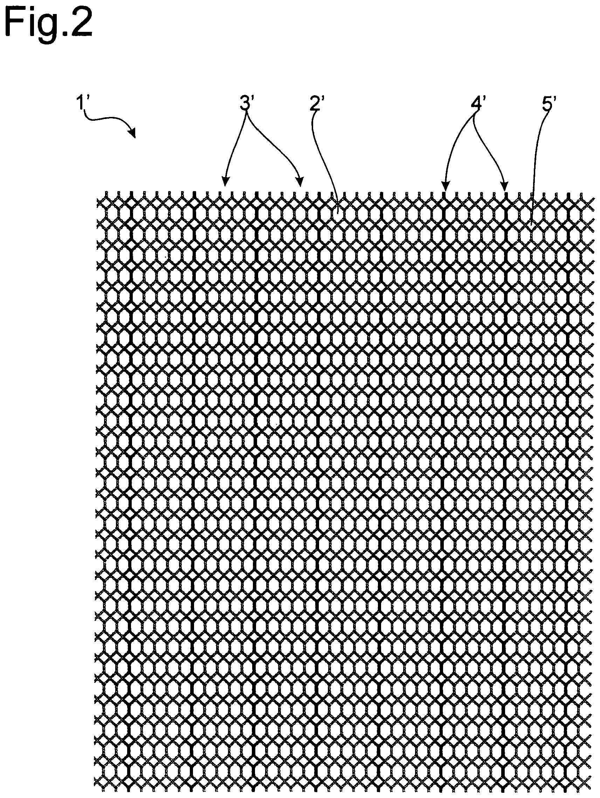

Bei der in der Zeichnung in Figur 2 dargestellten Ausführungsform der Erfindung ist ein thermofixiertes Herniennetz (1') als Einfachgewirk in Filetbindung ausgebildet. Das Gewirk hat eine netzartige Struktur, in der sich sechseckige Öffnungen (2') und rautenförmige Öffnungen (5') abwechseln. Die sechseckigen Öffnungen (2') sind in Längs- und Querreihen nebeneinander angeordnet. Die rautenförmigen Öffnungen (5') sind ebenfalls in Längs- und Querreihen angeordnet, wobei sie gegenüber den Reihen aus den sechseckigen Öffnungen versetzt sind und die verbleibenden Lücken ausfüllen. Die sechseckigen Öffnungen (2') besitzen eine lichte Maschenweite von ca. 4 x 6 mm. Die rautenförmigen Öffnungen besitzen eine lichte Öffnung von ca. 2 mm.

Das Gewirk ist wiederum streifenförmig ausgebildet, wobei sich breite Längsstreifen (3') mit schmalen Längsstreifen (4') regelmäßig abwechseln. Die breiten Längsstreifen bestehen aus monofilen Polypropylenfäden, die eine Fadenstärke von ca. 120 µm aufweisen. Demgegenüber bestehen die schmalen Längsstreifen aus monofilen Polypropylenfäden mit einer größeren Fadenstärke von ca. 150 µm. Die schmalen Längsstreifen (4') besitzen eine Breite von ca. 2,5 mm und die breiten Längsstreifen eine Breite von ca. 15 mm. Die schmalen Längsstreifen begrenzen jeweils eine Längsreihe von rautenförmigen Öffnungen, die durch einfache Längsstege miteinander verbunden sind. Das Verhältnis von rautenförmigen Öffnungen in Querrichtung, die aus stärkeren Fäden gebildet sind, zu denen, die aus schwächeren Fäden gebildet sind, liegt bei 1:4. Die stärkeren Fäden sind wiederum dunkel und bilden schmale in Längsrichtung verlaufende Guidelines.

Die farbigen Guidelines können auch unabhängig davon vorgesehen sein, ob es sich um Fäden größerer oder geringerer Stärke handelt. So können auch die Fäden geringerer Stärke, die neben den schmalen Streifen liegen, dunkler gefärbt sein als die anderen Fäden geringerer Fadenstärke, so dass sich breitere Farbstreifen ausbilden, ohne dass die eigentlichen Verstärkungsstreifen deshalb breiter gemacht werden müssen.

## Patentansprüche

1. Flächiges Implantat, insbesondere Herniennetz, in Form eines Öffnungen (2) aufweisenden textilen Netzes (1) aus nicht resorbierbaren monofilen Fäden, wobei das Netz (1) Fäden verschiedener Fadenstärke (3, 4) aufweist, wobei die Fäden in ihrer Grobstruktur parallel zueinander verlaufen, **dadurch gekennzeichnet, dass** die Fäden geringerer Stärke (3) zu den Fäden größerer Stärke (4) in einem Zahlenverhältnis von 10:1 bis 2:1 vorliegen.

2. Implantat nach Anspruch 1, **dadurch gekennzeichnet, dass** die Fäden verschiedener Stärke (3, 4) im wesentlichen nur in einer Längsrichtung des textilen Netzes verlaufen.

3. Implantat nach Anspruch 1 oder 2, **dadurch gekennzeichnet, dass** das Netz (1) ein Gewirk, insbesondere ein Gewirk mit rechts-links Bindung, ist.

4. Implantat nach einem der vorhergehenden Ansprüche, **dadurch gekennzeichnet, dass** die Fäden verschiedener Stärke (3, 4) in der gleichen textilen Bindung vorliegen und sich in der Bindung des Netzes abwechseln.

5. Implantat nach einem der vorhergehenden Ansprüche, **dadurch gekennzeichnet, dass** zwei verschiedene Fadenstärken vorgesehen sind.

6. Implantat nach einem der vorhergehenden Ansprüche, **dadurch gekennzeichnet, dass** jeweils mindestens zwei Fäden gleicher Fadenstärke nebeneinander verlaufen.

7. Implantat nach einem der vorhergehenden Ansprüche, **dadurch gekennzeichnet, dass** die Anzahl an Fäden mit geringerer Fadenstärke größer ist als die Anzahl der Fäden mit größerer Fadenstärke.

8. Implantat nach einem der vorhergehenden Ansprüche, **dadurch gekennzeichnet, dass** sich die Fäden mit größerer Fadenstärke (4) und die Fäden mit kleinerer Fadenstärke (3) in wiederholendem Rapport abwechseln.

9. Implantat nach einem der vorhergehenden Ansprüche, **dadurch gekennzeichnet, dass** die Fäden größerer Fadenstärke (4) um 15 bis 60 % dicker sind als die Fäden mit geringerer Fadenstärke (3).

10. Implantat nach einem der vorhergehenden Ansprüche, **dadurch gekennzeichnet, dass** die stärkeren Fäden einen Fadendurchmesser von 120 bis 200 µm aufweist und die dünneren Fäden 80 bis 150 µm aufweist.

11. Implantat nach einem der vorhergehenden Ansprüche, **dadurch gekennzeichnet, dass** das Netz ein Gewirk mit einer Filetbindung ist und die Öffnungen (2) rautenförmig ausgebildet sind.

12. Implantat nach einem der Ansprüche 1 bis 10, **dadurch gekennzeichnet, dass** das Netz ein Gewirk mit einer Filetbindung ist und die Öffnungen wabenförmig ausgebildet sind.

13. Implantat nach einem der Ansprüche 1 bis 10, **dadurch gekennzeichnet, dass** das Netz ein Gewirk mit einer Filetbindung ist und sich in Längsrichtung verlaufende wabenförmige Öffnungsreihen und rautenförmige Öffnungsreihen abwechseln, die in Längsrichtung gegeneinander versetzt sind.

14. Implantat nach einem der vorhergehenden Ansprüche, **dadurch gekennzeichnet, dass** es Verstärkungsstreifen aufweist, die durch thermische Verdichtung des Netzes ausgebildet sind.

15. Implantat nach Anspruch 14, **dadurch gekennzeichnet, dass** die thermische Verdichtung eine thermische Verbindung von sich überkreuzenden monofilen Fäden umfasst.

## Claims

1. Flat implant, in particular hernia mesh, in the form of a textile mesh (1) having openings (2) and made from non-resorbable monofilament yarns, wherein the mesh (1) has yarns of different yarn thicknesses (3, 4), wherein the yarns are running in parallel to each other in the coarse construction thereof,
**characterized in that**
the ratio of yarns having a lesser thickness (3) to yarns having a greater thickness (4) is in the order of 10:1 to 2:1.

2. Implant according to claim 1, **characterized in that** the yarns of different thicknesses (3, 4) basically only run in a longitudinal direction of the textile mesh.

3. Implant according to claim 1 or 2, **characterized in that** the mesh (1) is a warp-knitted textile, especially a warp-knitted textile having a single jersey construction.

4. Implant according to any of the preceding claims, **characterized in that** the yarns having different thicknesses (3, 4) are in a same textile construction and alternate with each other in the mesh construction.

5. Implant according to any of the preceding claims, **characterized in that** two different yarn thicknesses are provided.

6. Implant according to any of the preceding claims, **characterized in that** in each case at least two yarns of the same yarn thickness run adjacent to each other.

7. Implant according to any of the preceding claims, **characterized in that** the number of yarns having a lesser yarn thickness is higher than the number of yarns having a greater yarn thickness.

8. Implant according to any of the preceding claims, **characterized in that** the yarns having a greater yarn thickness (4) and the yarns having a lesser yarn thickness (3) alternate with each other in a repeating pattern.

9. Implant according to any of the preceding claims, **characterized in that** the yarns having a greater yarn thickness (4) are 15 to 60% thicker than the yarns having a lesser yarn thickness (3).

10. Implant according to any of the preceding claims, **characterized in that** the thicker yarns have a yarn diameter of 120 to 200 µm and the thinner yarns have a diameter of 80 to 150 µm.

11. Implant according to any of the preceding claims, **characterized in that** the mesh is a warp-knitted textile having a filet construction and the openings (2) are rhombic-shaped.

12. Implant according to any of claims 1 to 10, **characterized in that** the mesh is a warp-knitted textile having a filet construction and the openings are honeycomb-shaped.

13. Implant according to any of claims 1 to 10, **characterized in that** the mesh is a warp-knitted textile having a filet construction and honeycomb-shaped rows of openings alternate with rhombic-shaped rows of openings in the longitudinal direction and are staggered in relation to each other in the longitudinal direction.

14. Implant according to any of the preceding claims, **characterized in that** it has reinforcing strips formed by thermal consolidation of the mesh.

15. Implant according to claim 14, **characterized in that** the thermal consolidation comprises thermal bonding of intersecting monofilament yarns.

## Revendications

1. Implant en nappe, en particulier filet pour hernie, sous la forme d'un filet textile (1) présentant des ouvertures (2), constitué de fils monofilaments non résorbables, le filet (1) présentant des fils ayant des épaisseurs de fil différentes (3, 4), les fils, dans leur structure grossière, s'étendant parallèlement les uns aux autres, **caractérisé en ce que** les fils de plus faible épaisseur (3) sont présents par rapport aux fils de plus grande épaisseur (4) dans un rapport en nombre de 10:1 à 2:1.

2. Implant selon la revendication 1, **caractérisé en ce que** les fils d'épaisseurs différentes (3, 4) s'étendent essentiellement seulement dans une direction longitudinale du filet textile.

3. Implant selon la revendication 1 ou 2, **caractérisé en ce que** le filet (1) est un tricot, en particulier un tricot à armure à maillage gauche-droit.

4. Implant selon l'une quelconque des revendications précédentes, **caractérisé en ce que** les fils d'épaisseurs différentes (3, 4) sont présent dans la même armure textile et sont alternés dans l'armure du filet.

5. Implant selon l'une quelconque des revendications précédentes, **caractérisé en ce que** deux épaisseurs de fil différentes sous prévues.

6. Implant selon l'une quelconque des revendications précédentes, **caractérisé en ce qu'**à chaque fois au moins deux fils de même épaisseur de fil s'étendent l'un à côté de l'autre.

7. Implant selon l'une quelconque des revendications précédentes, **caractérisé en ce que** le nombre de fils ayant une plus faible épaisseur de fil est plus grand que le nombre des fils ayant une plus grande épaisseur de fil.

8. Implant selon l'une quelconque des revendications précédentes, **caractérisé en ce que** les fils ayant une plus grande épaisseur de fil (4) et les fils ayant une plus faible épaisseur de fil (3) sont alternés dans un rapport répétitif.

9. Implant selon l'une quelconque des revendications précédentes, **caractérisé en ce que** les fils ayant une plus grande épaisseur de fil (4) sont de 15 à 60 % plus épais que les fils ayant une plus faible épaisseur de fil (3).

10. Implant selon l'une quelconque des revendications précédentes, **caractérisé en ce que** les fils plus épais présentent un diamètre de fil de 120 à 200 µm et les fils plus minces présentent un diamètre de 80 à 150 pm.

11. Implant selon l'une quelconque des revendications précédentes, **caractérisé en ce que** le filet est un tricot ayant une armure en filet et les ouvertures (2) sont réalisées en forme de losange.

12. Implant selon l'une quelconque des revendications 1 à 10, **caractérisé en ce que** le filet est un tricot ayant une armure en filet et les ouvertures sont réalisées en forme de nid d'abeilles.

13. Implant selon l'une quelconque des revendications 1 à 10, **caractérisé en ce que** le filet est un tricot ayant une armure en filet et des rangées d'ouvertures en forme de nid d'abeilles s'étendant dans la direction longitudinale et des rangées d'ouvertures en forme de losange s'alternant et étant décalées les unes par rapport aux autres dans la direction longitudinale.

14. Implant selon l'une quelconque des revendications précédentes, **caractérisé en ce qu'**il présente des bandes de renforcement qui sont réalisées par compression thermique du filet.

15. Implant selon la revendication 14, **caractérisé en ce que** la compression thermique comprend une liaison thermique de fils monofilaments s'intersectant.
